# EUROPEAN PATENT APPLICATION

(11) **EP 1 762 226 A2**
(43) Date of publication of application: **14.03.2007**
(21) Application number: 06014342.7
(22) Date of filing: 11.07.2006
(51) Int. Cl.: A61K 9/06, A61K 31/522, A61K 31/167

(54) **Topical formulations in the form of gels containing acyclovir and lidocaine as active ingredients**

(30) Priority: 22.07.2005 IT MI20051417
(71) Applicant: Mipharm S.P.A., 20141 Milano (IT)
(72) Inventor: Miglio, Giuseppe, 20141 Milano (IT)
(74) Representative: Minoja, Fabrizio

(57) **Abstract**

Topical compositions of acyclovir and lidocaine in the form of gel comprising a polycarboxylic acid, a hydrophilic polymer, a hydrophobic polymer and an aqueous solvent.

## Description

The present invention relates to topical formulations in the form of gels containing acyclovir and lidocaine as active ingredients.

### TECHNOLOGICAL BACKGROUND

HSV is a pathogen often present both in immunocompetent and in immunocompromised patients, that causes diseases with different degree of severity. In the humans, HSV type 1 and 2 give rise respectively to frequent orofacial and genital lesions, although both viruses can infect any skin or mucosal area. HSV is associated to labial herpes, a widespread recurrent disease characterized by blisters which appear on red, swollen and painful areas which cicatrize without scars, but which tend to relapse.

Topical antiviral agents were used for the treatment of such infections, in particular idoxuridine in the early sixties, and acyclovir and peneiclovir more recently.

Labial Herpes can be either primary (inoculation) or secondary (recurrent). The lesions, whose number typically ranges from three to five in an area smaller than 100 mm², appear as maculae on the lips edge and evolve to painful papulae. The lesions ulcerate and form a crust within 72-96 hours and pain can be intense for 4-5 days. Acyclovir is an acyclic nucleoside analogue approved as antiviral in 1982 which is used for the systemic or topical treatment of Herpes simplex virus (HSV), chickenpox and Herpes zoster infections. Presently available topical formulations of acyclovir are creams and ointments. For the treatment of labial Herpes, cream formulations seem more effective than ointments. Acyclovir has a remarkable antiviral activity on HSV infections and poor toxicity.

Nevertheless, limitations to the topical use of acyclovir in the treatment of recurrent HSV skin infections have been documented. In fact, many common topical formulations do not ensure a reliable and adequate penetration of the active principle through the skin. Moreover, conventional formulations, such as creams, are not water-resistant, therefore they are progressively washed away by the saliva after application. Clinical tests have shown that an acyclovir cream is not much more effective than a placebo, the mean time of recovery from herpes lesions with the acyclovir cream being 4.3 days compared to 4.8 days with the placebo.

An acyclovir cream formulation in combination with lidocaine, one of the most frequently used local anaesthetics, was also disclosed (Int J Pharm Compound 2000 Jan/Feb;4(1):59). However, the analgesic effect of this combination seems to be of secondary importance in comparison with the acyclovir cream. This could be probably due to the fact that cream formulations do not allow optimal penetration of the active principles.

There is therefore a need for improved acyclovir topical formulations for the treatment of labial Herpes.

### Disclosure of the invention

It has now been found that topical formulations of acyclovir and lidocaine in the form of gels comprising a polycarboxylic acid surprisingly improve the skin penetration and therefore the therapeutic effect of the active ingredients compared with conventional cream formulations. Moreover, these formulations have improved water- and lick-resistance, which also contribute to increase effectiveness.

Accordingly, the invention provides topical compositions of acyclovir and lidocaine in the form of gel comprising a polycarboxylic acid, a hydrophilic polymer, a hydrophobic polymer and an aqueous solvent.

Examples of polycarboxylic acids that can be used according to the invention comprise citric, fumaric, succinic, malic and tartaric acids. Citric acid is particularly preferred.

The acids are present in the formulations of the invention in amounts ranging from 0.5 to 5%, preferably from 1 to 2% by weight.

The weight percentage of acyclovir can range from 0.5 to 10%, preferably from 2 to 7%, whereas the weight percentage of lidocaine can range from 0.5 to 3%, preferably from 1 to 2%.

Examples of suitable hydrophilic polymers comprise cellulose derivatives such as hydroxypropylmethylcellulose, hydroxypropylcellulose, hydroxyethylcellulose, carboxymethylcellulose, in weight percentage ranging from 1 to 5%, preferably hydroxypropylcellulose in percentage ranging from 2 to 5%.

Examples of hydrophobic polymers comprise polymethacrylates, aminoalkylmethacryl copolymers, ammonium methacrylate copolymers, polyvinylpyrrolidone, alkylacrylates and the like, in weight percentage ranging from 0.1% to 3%. Polymethacrylates commercially available under the trademark Eudragit E100 are preferred.

The aqueous solvent is preferably a water - ethanol mixture in ratios comprised between 1:1 and 3:1, respectively.

The invention is now illustrated in greater detail in the following examples.

### Example 1 - Formulation

| **Components** | **g/100 g** |
|---|---|
| Acyclovir | 5.00 |
| Lidocaine | 1.00 |
| Eudragit E 100 | 2.00 |
| Hydroxypropylcellulose (Klucel MF) | 4.00 |
| Citric acid | 1.00 |
| Purified water | 24.00 |
| Ethanol 96° | 63.00 |
| Total | 100.00 |

### Example 2 - Skin penetration tests

Skin penetration of the formulation of Example 1 was evaluated with the "Franz's Cells Test", according to a well-known experimental protocol, using human skin isolated from six women of age ranging from 31 to 51 and subjected to aesthetic surgery. The "Stratum Corneum-Epidermis" (SCE) membranes were obtained from the skin. The formulation of Example 1 was compared to a commercial cream formulation containing 5% of acyclovir. The results reported in Table 1 show that the amount of acyclovir released from the formulation of the invention is four-fold greater than the amount released from the cream formulation.

**Table 1: Acyclovir skin penetration in comparison with a commercial cream formulation**

| **Subject** | **Formulation of Example 1** | **Acyclovir commercial cream** |
|---|---|---|
| **A** | *21.6* | *3.8* |
| **B** | *30.4* | *5.6* |
| **C** | *17.5* | *4.2* |
| **D** | *24.3* | *3.7* |
| **E** | *28.7* | *5.1* |
| F | 19.4 | 6.3 |
| **Mean** | **23.6** | **4.8** |
| **±SD** | *5.2* | *1.1* |

## Claims

1. Topical compositions of acyclovir and lidocaine in the form of gels comprising a polycarboxylic acid, a hydrophilic polymer, a hydrophobic polymer and an aqueous solvent.

2. Compositions as claimed in claim 1, in which the polycarboxylic acid is citric acid.

3. Compositions as claimed in claim 1 or 2 containing the acids in weight percentage ranging from 0.5 to 5%, preferably from 1 to 2%.

4. Compositions as claimed in any one of claims 1 to 3 containing acyclovir in weight percentage ranging from 0.5 to 10%, preferably from 2 to 7%, and lidocaine in weight percentage ranging from 0.5 to 3%, preferably from 1 to 2%.

5. Compositions as claimed in any one of claims 1 to 4 in which the hydrophilic polymers are selected from hydroxypropylmethylcellulose, hydroxypropylcellulose, hydroxyethylcellulose, carboxymethylcellulose.

6. Compositions as claimed in claim 5 containing the hydrophilic polymers in weight percentage ranging from 1 to 5%.

7. Compositions as claimed in claim 5 in which the hydrophilic polymer is hydroxypropylcellulose.

8. Compositions as claimed in any one of claims from 1 to 7 in which the hydrophobic polymers are selected from polymethacrylates, aminoalkylmetacryl copolymers, ammonium methacrylate copolymers, polyvinylpyrrolidone, alkylacrylates and the like.

9. Compositions as claimed in claim 8 containing the hydrophobic polymers in weight percentage ranging from 0.1 % to 3%.

10. Compositions as claimed in claim 8 in which the hydrophobic polymers are commercially available Eudragit E100 polymethacrylates.

11. Compositions as claimed in any one of the above claims in which the aqueous solvent is a mixture of water and ethanol in ratios comprised between 1:1 and 3:1.
